# EUROPÄISCHE PATENTSCHRIFT

(11) **EP 1 305 003 B1**
(45) Veröffentlichungstag und Bekanntmachung des Hinweises auf die Patenterteilung: **03.11.2004**
(21) Anmeldenummer: 01971796.6
(22) Anmeldetag: 27.07.2001
(51) Int. Cl.: A61K 7/06, A61K 31/12, A61P 17/14

(54) **vERWENDUNG EINER BIOCHINONE ENTHALTENDEN WIRKSTOFFKOMBINATION ZUR HERSTELLUNG KOSMETISCHER ODER DERMATOLOGISCHER ZUBEREITUNGEN ZUR BEHANDLUNG UND PROPHYLAXE VON KOPFSCHUPPEN**
USE OF A COMBINATION OF ACTIVE SUBSTANCES COMPRISING BIOQUINONES FOR THE MANUFACTURE OF COSMETIC OR DERMATOLOGICAL COMPOSITIONS FOR THE TREATMENT OR THE PREVENTION OF DANDRUFF
UTILISATION D'UNE COMBINAISON D'AGENTS ACTIVS CONTENATN DES BIOQUINONES POUR LA PREPARATION D'UNE COMPOSITION DOSMETIQUES OU DERMATOLOGIQUE POUR LE TRAITEMENT OU LA PREVENTION DES PELLICULES

(30) Priorität: 28.07.2000 DE 10036799
(43) Veröffentlichungstag der Anmeldung: 02.05.2003
(73) Patentinhaber: GS DEVELOPMENT AB, 213 75 Malmö (SE)
(72) Erfinder: HOPPE, Udo, 24598 Heidmühlen (DE); MEI, Weiping, 22457 Hamburg (DE); SAUERMANN, Gerhard, 24649 Wiemersdorf (DE)
(74) Vertreter: UEXKÜLL & STOLBERG
(86) Internationale Anmeldenummer: PCT/EP2001/008728
(87) Internationale Veröffentlichungsnummer: WO 2002/009664

(56) Entgegenhaltungen:
- WO-A-92/02225
- WO-A-97/02041
- US-A- 5 407 944
- PATENT ABSTRACTS OF JAPAN vol. 008, no. 098 (C-221), 9. Mai 1984 (1984-05-09) & JP 59 013719 A (SHISEIDO KK;OTHERS: 01), 24. Januar 1984 (1984-01-24)

## Beschreibung

Gegenstand der Erfindung ist die Verwendung einer Biochinone enthaltenden Wirkstoffkombination zur Herstellung kosmetischer oder dermatologischer Zubereitungen, die gegen Schuppen wirksam ist.

Bekanntlich entspricht das Haarwachstum einem Zyklus. Papillenhaar wird das Haar genannt, das sich in der Wachstumsperiode befindet, die auch anagene Phase oder Anagenphase genannt wird. In dieser Phase ist das Haar mit seiner Papille in der Haut verankert. Etwa 80% der Kopfhaare befinden sich etwa 3 bis 5 Jahre lang in der Anagenphase. In einer sich anschließenden Übergangsphase (Katagenphase) wandert das Haar etwa 2 Wochen lang an die Hautoberfläche und bleibt dann etwa 3 bis 4 Monate lang in einem Ruhestadium (Telogenphase) bis es schließlich ausfällt.

Ein über das normale Maß hinausgehender Haarausfall wird zumeist als schwere kosmetische Störung angesehen, ebenso wie andere Wachstumsstörungen der Haare. Daher wurden schon viele Mittel zur Behandlung von Haarausfall und Glatzenbildung sowie Haarwuchsmittel vorgeschlagen, die das Wachstum der Haare erhalten oder fördern sollen.

Aus dem deutschen Patent 12 96 310 und aus der PCT-Schrift WO 85/04577 sind auch schon Stoffe bekannt, die eine Verlängerung der Wachstumsperiode bzw. eine Verlängerung der anagenen Phase bewirken. Es handelt sich dabei um eine substituierte Aminosäure oder um substituierte Pyridylpyrimidine.

Aus dem US-Patent 4,654,373 ist weiterhin die topische Anwendung der Verbindung Coenzym Q₁₀ zur Prävention dystrophischer oder dysmetabolischer Zustände der Haut oder ihrer Anhänge bekannt.

In der PCT-Schrift WO 88/03015 sind wässrige Zubereitungen beschrieben, die Ubidecarenon (Ubichinon-10) und bestimmte amphipatische Verbindungen enthalten, die mit dem Ubidecarenon micellare und liposominale Aggregate bilden. Bei Anwendung auf der Haut sollen verschiedene kosmetische Effekte erhalten werden, darunter auch die Anregung des Haarwuchses.

Aus EP-A-0 100 915 und JP 59 013 719 ist ein Haarwuchsmittel bekannt, das Ubichinone (z.B. Coenzym Qₙ n=7-10) enthält und gegebenenfalls auch zusätzlich in der Haut, peripher, wirksame Vasodilatoren wie Carproniumchlorid, Vitamin-E-nicotinat und Benzylnicotinat.

Aus der Literatur sind weiterhin haarwuchsfördernde Antihypertensiva bekannt, die zu der Gruppe der Kaliumkanalöffner gehören. Ein solcher, sehr bekannter Wirkstoff ist Minoxidil. Kaliumkanalöffner gehören, neben anderen Stoffgruppen, zu den Vasodilatoren.

Auch aus dem US-Patent 4,139,619 und aus EP-A-0 188 793 sind Haarwuchsmittel mit Minoxidil als Wirkstoff bekannt, die topisch angewendet werden.

Aus der Literatur (Merck Index, 12. Auflage, Abstract No. 4125) ist weiterhin der 5-aipha-Reduktasehemmer Finasterid (INN) bekannt, der im Tierversuch Wirkungen hinsichtlich des Haarwachstums haben soll.

Aus der Literatur ist ferner eine Zubereitung bekannt, die Coenzym Q10, Acetylcamitin und einen 5-alpha-Reduktasehemmer enthält (WO 97/02041) und für die Behandlung androgener bzw. seborrhoeischer Alopecia geeignet ist.

US 5,407, 944 und WO 92/02225 offenbaren die Verwendung von Kaliumkanalöffnern alleine oder in Kombination mit anderen haarwuchsfördernden Wirkstoffen, z.B. dem 5-alpha-Reduktasehemmer Finasterid, zur Förderung des Haarwuchses.

In EP-0 309 086 wird die Verwendung ungesättigter Fettsäuren, z.B. von Gammalinolensäure, zur Modulation des Haarwachstums offenbart.

JP 63 277604 beschreibt eine kosmetische Züsammensetzung die Alphalinolensäure oder ihre Derivate, z.B. Alpha-linolensäure DL alpha-Tocopherol gegebenenfalls züsammen mit gamma-Linolensäure, enthält und verschiedenste positive Auswirkungen auf die Haut hat, unter anderem auch die Unterdrückung von Schuppen.

In PAJ Abstract von JP4193821 wird ein Haarpflegemittel beschrieben, welches Minoxidil enthält, und dem ein reduzierende Wirkung auf Kopfschuppen zugesprochen werden.

In WPI Derwent Abstract (AN 2000-199599) von JP2000007534 wird ein Haarpflegemittel beschrieben, welches Finasteride enthält, und eine Alopecia vorbeugende Wirkung hat, sowie Kopfschuppen und Kopfjucken vorbeugt.

Alle diese Dokumente konnten jedoch nicht den Weg zu der vorliegenden Erfindung bahnen.

Als eine unangenehme kosmetische Störung der Kopfhaut werden Schuppen (Kopfschuppen) angesehen. Zu ihrer Behandlung wurden schon viele Vorschläge gemacht.

Wünschenswert sind daher insbesondere topische kosmetische Zubereitungen, die sich nicht nur auf das Haarwachstum günstig auswirken, sondern auch die Kopfhaut pflegen und die Schuppenbildung verringern oder verhindern.

Bekannte Haarbehandlungsmittel haben oft Nachteile. Häufig ist ihre Wirkung nicht zufriedentellend oder sie sind gesundheitlich nicht unbedenklich, gerade bei ständiger Anwendung.

Die Aufgabe der Erfindung besteht daher darin, bessere Mittel zur Beeinflussung des Haarwachstums und zur Prophylaxe und Behandlung von Schuppen (Kopfschuppen) bereitzustellen.

Diese Aufgaben werden erfindungsgemäß gelöst.

Gegenstand der Erfindung ist die Verwendung einer Verbindung oder mehrerer Verbindungen aus der Gruppe der Biochinone
a) in Kombination mit einer Verbindung oder mehreren Verbindungen aus der Gruppe der Kaliumkanalöffner und
b) in Kombination mit einer Verbindung oder mehreren Verbindungen aus der Gruppe der 5-alpha-Reduktasehemmer, zur Herstellung kosmetischer oder dermatologischer Zubereitungen für die Behandlung der Kopfhaut und der Haare zur Verlängerung der anagenen Phase und/oder zur Behandlung und Prophylaxe von Kopfschuppen gegebenenfalls jeweils unter zusätzlicher Verwendung von einer Verbindung oder mehreren Verbindungen aus der Gruppe, gebildet von Carnitin, Arginin, Bernsteinsäure, Folsäure, konjugierter Fettsäure und jeweils deren Derivaten, sowie Antioxidantien.

Bevorzugt werden erfindungsgemäße Kombinationen oder Verwendungen für die die folgenden Wirkstoffe kombiniert bzw. verwendet werden:
a) Ubichinone, insbesondere Coenzym Q-10, und Minoxidil, oder
b) Ubichinone, insbesondere Coenzym Q-10, und Finasterid und/oder Gammalinolensäure, oder
c) Ubichinone, insbesondere Coenzym Q-10, und Minoxidil und Finasterid und/oder Gammalinolensäure,
gegebenenfalls jeweils unter zusätzlicher Verwendung von einer Verbindung oder mehreren Verbindungen aus der Gruppe, gebildet con Carnitin, Arginin, Bernsteinsäure, Folsäure, konjugierter Fettsäure und jeweils deren Derivaten, sowie Antioxidantien, vorzugsweise aber unter zusätzlicher Verwendung von Carnitin und/oder konjugierten Fettsäuren, insbesondere konjugierter Linolsäure, oder jeweils deren Derivaten.

Es kann die folgenden Wirkstoff-Kombinationen vorliegen (mit mindestens jeweils einer Verbindung aus der gewünschten Wirkstoffgruppe)::
3) Biochinon und Kaliumkanalöffner und 5-alpha-Reduktasehemmer.

Die erfindungsgemäßen Mittel werden vorzugsweise topisch angewendet.

Als Biochinone werden unterschiedlich substituierte prenylierte Chinone bezeichnet, die in Menschen, Tieren und Pflanzen vorkommen.

Bevorzugte Biochinone sind Ubichinone, Plastochinone und Bovichinone, insbesondere aber Ubichinone.

Gut geeignete Ubichinone zeichnen sich durch die Strukturformel 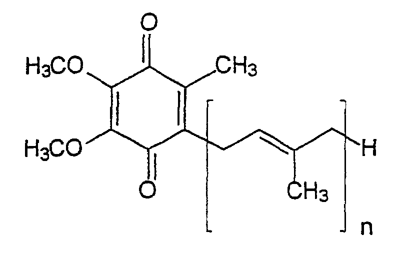 aus (n = 1-10) und stellen die am weitesten verbreiteten u. damit am besten untersuchten Biochinone dar. Ubichinone werden je nach Zahl der in der Seitenkette verknüpften Isopren-Einheiten als Q-1, Q-2, Q-3 usw. oder nach Anzahl der C-Atome als U-5, U-10, U-15 usw. bezeichnet. Sie treten bevorzugt mit bestimmten Kettenlängen auf, z. B. in einigen Mikroorganismen u. Hefen mit n=6. Bei den meisten Säugetieren einschließlich des Menschen überwiegt Q-10.

Coenzym Q-10 wird bevorzugt. Es ist durch folgende Strukturformel gekennzeichnet

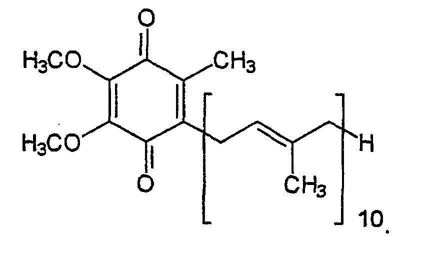

Ubichinone dienen den Organismen als Elektronenüberträger in der Atmungskette. Sie befinden sich in den Mitochondrien wo sie die cyclische Oxidation und Reduktion der Substrate des Citronensäure-Cyclus ermöglichen.

Gut geeignete Plastochinone weisen die allgemeine Strukturformel (n = 1-10) 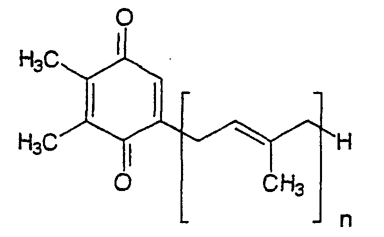 auf. Sie können aus Chloroplasten isoliert werden und spielen als Redoxsubstrate in der Photosynthese beim cyclischen und nichtcyclischen Elektronentransport eine Rolle, wobei sie reversibel in die entsprechenden Hydrochinone (Plastochinol) übergehen. Plastochinone unterscheiden sich in der Anzahl n der Isopren-Reste und werden endsprechend bezeichnet, z. B. PQ-9 (n=9). Femer existieren andere Plastochinone mit unterschiedlichen Substituenten am Chinon-Ring.

Erfindungsgemäß bevorzugtes Biochinon ist das Coenzym Q-10.

Es ist vorteilhaft, in den fertigen Zubereitungen Konzentrationen von 0,000001 - 10 Gew.-%, insbesondere 0,001 -1, an einem oder mehreren Biochinonen, bevorzugt Coenzym Q-10, zu wählen, jeweils bezogen auf das Gesamtgewicht der Zubereitungen.

Eine Vielzahl von Kaliumkanalöffnern ist in der Literatur beschrieben.

Erfindungsgemäß gut geeignet sind die folgenden Wirkstoffe:
- Minoxidil
- Pinacidil
- Diazoxid
- Cromakalim
- Rilmakalim
- Nicorandil
- Flupirtine

- KRN2391
- P-1075
- ZD6169
- RP-49,356
- YM934
- MCC-134 und
- SKP 450.

Besonders bevorzugt werden die folgenden Wirkstoffe:
- Minoxidil, z.B. Minoxidilsulfat
- Pinacidil
- Diazoxid
- Cromakalim
- Rilmakalim
- Nicorandil
- Flupirtine,
insbesondere aber Minoxidil und/oder Pinacidil.

Kaliumkanalöffner sind vorzugsweise in Mengen von 0,01 bis 20 Gew.-%, besonders bevorzugt 0,1 bis 5 Gew.-%, insbesondere 1 bis 3 Gew.-%, jeweils bezogen auf die gesamte Zubereitung in den erfindungsgemäßen kosmetischen und dermatologischen Zubereitungen enthalten.

Das Verhältnis der Gewichtsmengen der Kombinations-Wirkstoffe Biochinone/Kaliumkanalöffner zueinander kann in den Zubereitungen stark variieren. Beispielsweise kann es 1/10 bis 10/1, oder 5/1 bis 1/5 betragen. Vorzugsweise kann es aber auch 1/2 bis 2/1 und insbesondere 1/1 betragen.

5-alpha-Reduktasehemmer bzw. Reduktase-Inhibitoren sind in der Literatur beschrieben. Sie inhibieren ein Enzym, das die Umwandlung von Testosteron in das potentere Androgen 5-alpha-Dihydrotestosteron bewirkt.

Geeignete 5-alpha-Reduktasehemmer sind beispielsweise steroidale aber auch nichtsteroidale 5-alpha-Rekuktasehemmer wie in der Literatur beschrieben (W. Chen. Et al, Dermatologie, **1996,** 193:177-184).

Steroidale 5-alpha-Reduktasehemmer sind z.B. Finasterid, Turosterid, MK-434, MK-963, Epristerid und MK-386.

Nichtsteroidale 5-alpha-Reduktasehemmer sind beispielsweise ONO-3805, LY191704, FK 143, polyungesättigte Fettsäuren, Zink-Ionen, z.B. die wasserlöslichen Salze anorganischer Säuren, Catechine, z.B. Catechin, Epicatechin, Extrakte des Tees, z.B. des Grünen oder Schwarzen Tees, Epicatechin-3-gallat oder Epigallocatechin-3-gallat.

Gut geeignete polyungesättigte Fettsäuren können beispielsweise jeweils bis zu 24, vorzugsweise bis zu 18, insbesondere bis zu 12 Kohlenstoffatome besitzen und z.B. geradkettige oder verzweigte Alkyl-Monocarbonsäuren oder Cycloalkyl-Monocarbonsäuren sein. Sie können beispielsweise zwei bis sechs Mehrfachbindungen, insbesondere Doppelbindungen besitzen.

Besonders bevorzugt werden Gammalinolensäure (GLA), Zinksalze, z.B. Zinkchlorid, Tee- und Grüntee-Extrakte, Catechine, z.B. Epicatechin-3-gallat und/oder Epigallocatechin-3-gallat.

Bevorzugt wird Finasterid (17β-(N-tert-butylcarbamoyl)-4-aza-5α-androstan-1-en-3-on) wie vorstehend schon zitiert.
5-alpha-Reduktasehemmer sind vorzugsweise Mengen von 0,01 bis 10 Gew.-%, besonders bevorzugt 0,1 bis 1 Gew.-%, insbesondere 0,4 bis 0,6 Gew.-%, jeweils bezogen auf die gesamte Zubereitung in den erfindungsgemäßen kosmetischen und dermatologischen Zubereitungen enthalten.

Das Verhältnis der Gewichtsmengen der Kombinations-Wirkstoffe Biochinone/5-alpha-Reduktasehemmer kann in den Zubereitungen stark variieren. Beispielsweise kann es 1/10 bis 1011, oder 5/1 bis 1/5 betragen. Vorzugsweise kann es aber auch 1/2 bis 2/1 und insbesondere 1/1 betragen.

Bevorzugt werden Zubereitungen die folgende Wirkstoffkombinationen umfassen:
1) Q-10 und Minoxidil
2) Q-10 und Finasterid und/oder Gammalinolensäure
3) Q-10 und Minoxidil und Finasterid und/oder Gammalinolensäure

Vorzugsweise werden auch dafür die vorstehend genannten Gewichtsverhältnisse verwendet.

Den Zubereitungen, die mindestens ein Biochinon und in Kombination mindestens einen Kaliumkanalöffner und einen 5-alpha-Reduktasehemmer als Wirkstoffe enthalten, können vorzugsweise weitere Wirkstoffe wie Carnitin, Arginin, Bernsteinsäure, konjugierte Fettsäure und/oder Folsäure bzw. auch jeweils deren Derivate und/oder gegebenenfalls eine Verbindung oder mehrere Verbindungen aus der Gruppe der Antioxidantien zugesetzt werden, z.B. um die Wirkung zu verbessern.

Geeignete Derivate des Camitins sind beispielsweise O-Acylcarnitine mit geradkettigen oder verzweigten C₁ - C₁₂-Alkylgruppen des Alkylcarbonylrestes (Acylrestes). Acetylcamitin und dessen Derivate, z.B. wie nachstehend angegeben, werden bevorzugt Camitin und die Acylcarnitine können auch als Salze, Säureadditionssalze, Ester oder Amide verwendet werden.

Bevorzugte Salze sind wasserlösliche Salze, z.B. Natrium-, Kalium- und Ammoniumsalze. Dies gilt auch für die Säureadditionssalze. Geeignete Säureadditionssalze werden mit anorganischen und organischen Säuren erhalten. Bevorzugt werden die Hydrochloride, Sulfate, Acetate, Caprylate oder Zitrate.

Geeignete Ester sind z.B. solche, die mit kurzkettigen, mittelkettigen oder langkettigen Alkoholen erhalten werden, vorzugsweise mono-Alkohole, insbesondere aber Methanol, Ethanol oder Propanol. Bevorzugt werden die Ethylester.

Bevorzugte Amide sind kurz- oder mittelkettige oder langkettige mono- und di-Alkylamide.

Alkyle der vorstehenden Substituenten enthalten z.B. bis zu 20, vorzugsweise bis zu 6 Kohlenstoffatome, insbesondere ein oder zwei Kohlenstoffatome.

Carnitin und/oder seine Derivate sind in den erfindungsgemäßen Zubereitungen bevorzugt in Mengen von 0,00001 bis 10 Gew.-%, insbesondere 0,01 bis 1,5 Gew.-%, jeweils bezogen auf das Gesamtgewicht der Zubereitungen enthalten.

Arginin kann als Racemat oder in optisch aktiver Form (D- oder L-) vorliegen. Bevorzugt werden L-Arginin und/oder dessen Derivate.

Geeignete Derivate des Arginins sind beispielsweise dessen Salze; Säureadditionssalze Ester oder Amide.

Bevorzugte Salze von Arginin sind wasserlösliche Salze, z. B. Natrium-, Kalium- und Ammoniumsalze. Dies gilt auch für die Säureadditionssalze. Geeignete Säureadditionssalze werden mit anorganischen oder organischen Säuren erhalten. Bevorzugt werden die Hydrochloride, Sulfate, Acetate, Caprylate oder Zitrate.

Geeignete Ester des Arginins sind z.B. solche, die mit kurzkettigen oder mittelkettigen oder langkettigen Alkoholen erhalten werden, vorzugsweise mono-Alkohole, insbesondere aber Methanol, Ethanol oder Propanol. Bevorzugt werden die Ethylester.

Bevorzugte Amide sind kurz- oder mittelkettige oder langkettige mono- und di-Alkylamide.

Alkyle der vorstehenden Substituenten enthalten z.B. bis zu 20, vorzugsweise bis zu 6 Kohlenstoffatome, insbesondere ein oder zwei Kohlenstoffatome.

Arginin und seine Derivate zeichnen sich auch durch ein besonders gutes Hautpenetrationsvermögen aus.

Arginin und seine Derivate sind vorzugsweise in Mengen von 0,01 bis 30 Gew.-%, besonders bevorzugt 0,01 bis 10 Gew.-%, insbesondere 0,1 - 7,5 Gew.-%, jeweils bezogen auf die gesamte Zubereitung, in den erfindungsgemäßen kosmetischen und dermatologischen Zubereitungen enthalten.

Geeignete Derivate der Bemsteinsäure sind beispielsweise die Succinate, d.h. die Bemsteinsäure-Ester und -Salze, sowie die jeweiligen Hydrogensuccinate und auch die Säureadditionssalze, aber auch Bernsteinsäure-Amide oder die entsprechenden Hydrogen-Amide.

Bevorzugte Salze, Säureadditionssalze oder Ester sind solche, wie sie schon für die Argininderivate beschrieben wurden.

Bevorzugt wird Dinatriumsuccinat.

Bernsteinsäure und/oder ihre Derivate sind vorzugsweise in Mengen von 0,001 bis 30 Gew.-%, besonders bevorzugt 0,01 bis 20 Gew.-%, besonders bevorzugt 0,01 bis 10 Gew.-%, insbesondere 0,1 - 7,5 Gew.-%, jeweils bezogen auf die gesamte Zubereitung, in den erfindungsgemäßen kosmetischen und dermatologischen Zubereitungen enthalten.

Geeignete Derivate der Folsäure sind beispielsweise deren Salze, Säureadditionssalze oder Ester oder Amide. Bevorzugt werden solche Salze, Säureadditionssalze, Ester oder Amide wie sie schon für die Argininderivate beschrieben wurden.

Vorzugsweise wird Folsäure verwendet.

Folsäure und/oder ihre Derivate sind in den erfindungsgemäßen Zubereitungen bevorzugt in Mengen von 0,0001 bis 5 Gew.-%, insbesondere 0,01 bis 1,5 Gew.-%, jeweils bezogen auf das Gesamtgewicht der Zubereitungen enthalten.

Weitere wichtige Komponenten, die neben Carnitin, Arginin, Bemsteinsäure, Folsäure geeignet sind z.B. den Energiestoffwechsel der Haarwurzeln zu verbessern, sind konjugierte Fettsäuren, d.h. Monocarbonsäuren mit mindestens zwei konjugierten Mehrfachbindungen, insbesondere Doppelbindungen und deren Derivate. Sie werden hier auch "CFA" genannt. Gut geeignet sind alle geometrischen isomeren Formen und stellungsisomeren Formen sowie die Gemische solcher Verbindungen sowie deren Derivate, beispielsweise die Salze, Ester oder Amide.

Solche konjugierten Fettsäuren sind bekannt und nach bekannten Verfahren erhältlich, beispielsweise durch alkalische Isomerisierung der entsprechenden Fettsäuren mit isolierten Mehrfachbindungen bzw. Doppelbindungen.

Gut geeignete Fettsäuren können beispielsweise jeweils bis zu 24, vorzugsweise bis zu 18, insbesondere bis zu 12 Kohlenstoffatome besitzen und z.B. geradkettige oder verzweigte Alkyl-Monocarbonsäuren oder Cycloalkyl-Monocarbonsäuren sein. Sie können beispielsweise 2 bis 6 konjugierte Mehrfachbindungen, insbesondere Doppelbindungen besitzen.

Bevorzugte Salze sind wasserlösliche Salze, z.B. Natrium-, Kalium- und Ammoniumsalze.

Geeignete Ester sind z.B. solche, die mit kurzkettigen, mittelkettigen oder langkettigen Alkoholen erhalten werden, vorzugsweise Mono-Alkohole, insbesondere aber Methanol, Ethanol oder Propanol. Bevorzugt werden die Ethylester.

Bevorzugte Amide sind kurz- oder mittelkettige oder langkettige mono- und di-Alkylamide.

Alkyle der vorstehenden Substituenten erhalten z.B. bis zu 20, vorzugsweise bis zu 6 Kohlenstoffatome, insbesondere ein oder zwei Kohlenstoffatome.

Eine bevorzugte CFA, die z.B. den Energiestoffwechsel der Haarwurzel verbessert, ist neben Carnitin, Arginin, Bernsteinsäure, Folsäure die konjugierte Linolsäure, auch "CLA" genannt, in allen ihren geometrischen isomeren Formen und stellungsisomeren Formen sowie den Gemischen solcher Verbindungen sowie ihren Derivaten, insbesondere wie vorstehend beschrieben.

Linolsäure (cis, cis-9,12-Octadecadiensäure) hat keine konjugierten Doppelbindungen. Distelöl und Sonnenblumenöl besitzen einen hohen Anteil an dieser Säure. Beispielsweise aus der Linolsäure dieser Rohstoffe erhält man durch alkalische Isomerisierung in bekannter Weise die konjugierten Verbindungen. Ein bevorzugtes Isomerengemisch ist auch in der Literatur beschrieben (Lipids, vol. 34, Nr. 9 (1999) S. 997-1000, Tabelle 1). Bevorzugt liegen die konjugierten Doppelbindungen der CAFs im Bereich der Kohlenstoffatome 9 bis 12.

CFAs oder CLA und/oder die Derivate sind in den erfindungsgemäßen Zubereitungen bevorzugt in Mengen von 0,0001 bis 5 Gew.-%, insbesondere 0,01 bis 1,5 Gew.-%, jeweils bezogen auf das Gesamtgewicht der Zubereitungen enthalten.
Vorzugsweise werden Camitin und/oder seine Derivate in Kombination mit CFAs und/oder deren Derivaten verwendet, insbesondere in den jeweils angegebenen Gewichtsmengen. Das Verhältnis der Gewichtsmengen dieser Kombinations-Wirkstoffe Carnitin/CFAs kann in den Zubereitungen stark variieren. Beispielsweise kann es 1/10 bis 10/1, oder 5/1 bis 1/5 betragen. Vorzugsweise kann es aber auch 1 / 2 bis 2/1 und insbesondere 1/1 betragen.

Erfindungsgemäß wird die Bildung von Kopfschuppen verhindert oder stark vermindert.

Zur Anwendung werden die erfindungsgemäßen Zubereitungen vorzugsweise direkt auf die Kopfhaut aufgebracht, und zwar in der für solche Mittel bekannten Weise, beispielsweise zweimal täglich.

Geeignet sind z.B. Lösungen, Gele, Salben, Suspensionen oder Emulsionen wie Cremes oder Lotionen mit einem Gehalt an den erfindungsgemäßen Wirkstoffen.

Auch Haarbehandlungsmittel mit einem Gehalt an den erfindungsgemäßen Wirkstoffen sind geeignet, insbesondere solche, die im Haar verbleiben oder mit längerer Einwirkzeit verwendet werden. Auch auf diese Weise gelangen die Wirkstoffe in oder auf die Kopfhaut oder in den Bereich der Haarwurzeln. Haarbehandlungsmittel, die nur kurze Zeit mit der Haut oder den Haaren in Berührung kommen, z.B. Shampoos, können beispielsweise höhere Wirkstoffanteile haben.

Haarbehandlungsmittel sind beispielsweise Haarwaschmittel, Haarpflegemittel wie Haarwässer, Frisierhilfsmittel, Haarspülungen, Haarkuren, Kurpackungen, Haarfestiger wie Schaumfestiger, Haarspray, Haarlack, Haarverformungsmittel und Haarfärbemittel.

Erfindungsgemäße kosmetische und gegebenenfalls dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe oder auch ein Aerosol darstellen. Es ist auch erfindungsgemäße vorteilhaft, ein oder mehrere Biochinone in verkapselter Form darzureichen, z.B. in Kollagenmatrices und anderen üblichen Verkapselungsmaterialien, z.B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Insbesondere können erfindungsgemäß verwendete Biochinone auch mit Antioxidantien, darunter auch Radikalfängern, kombiniert werden.

Vorteilhaft werden solche Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. Linolsäure, Ölsäure), Tocopherole und Derivate (z.B. Vitamin-E-acetat), Vitamin A und Derivate (Vitamin-A-palmitat) sowie Koniferylbenzoat des Benzoeharzes, Flavonoide, z.B. alpha-Glucosylrutin, Rutinsäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Sesamol, Sesamolin, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die zusätzlichen Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die zusätzlichen Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Erfindungsgemäß sind Emulsionen vorteilhafte Verkörperung der Erfindung und enthalten z.B. die genannten Fette, Öle, Wachse und anderen Fettkörper, sowie Wasser und einen Emulgator, wie er üblicherweise für einen solchen Typ der Formulierung verwendet wird.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, lsooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, lsoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrigen erfindungsgemäßen Zubereitungen bzw. die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder weiche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole® beispielsweise Carbopole® der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Im technischen Sinne werden unter Gelen verstanden: Relativ formbeständige, leicht verformbare disperse Systeme aus zumindest zwei Komponenten, welche in der Regel aus einem - meist festen - kolloid zerteilten Stoff aus langkettigen Molekülgruppierungen (z.B. Gelatine, Kieselsäure, Polysaccharide) als Gerüstbildner und einem flüssigen Dispersionsmittel (z.B. Wasser) bestehen. Der kolloidal zerteilte Stoff wird oft als Verdickungs- oder Geliermittel bezeichnet. Er bildet ein räumliches Netzwerk im Dispersionsmittel, wobei einzelne kolloidal vorliegende Partikel über elektrostatische Wechselwirkung miteinander mehr oder weniger fest verknüpft sein können. Das Dispersionsmittel, welches das Netzwerk umgibt, zeichnet sich durch elektrostatische Affinität zum Geliermittel aus, d.h., ein vorwiegend polares (insbesondere: hydrophiles) Geliermittel geliert vorzugsweise ein polares Dispersionsmittel (insbesondere: Wasser), wohingegen ein vorwiegend unpolares Geliermittel vorzugsweise unpolare Dispersionsmittel geliert.

Starke elektrostatische Wechselwirkungen, welche beispielsweise in Wasserstoffbrükkenbindungen zwischen Geliermittel und Dispersionsmittel, aber auch zwischen Dispersionsmittelmolekülen untereinander verwirklicht sind, können zu starker Vernetzung auch des Dispersionsmittels führen. Hydrogele können zu fast 100 % aus Wasser bestehen (neben beispielsweise ca. 0,2 - 1,0 % eines Geliermittels) und dabei durchaus feste Konsistenz besitzen. Der Wasseranteil liegt dabei in eisähnlichen Strukturelementen vor, so daß Gele daher ihrer Namensherkunft [aus lat. "gelatum" = "Gefrorenes" über den alchimistischen Ausdruck "gelatina" (16. Jhdt.) für nhdt. "Gelatine"] durchaus gerecht werden.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Enthalten die erfindungsgemäßen Zubereitungen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester, - 2,4,6-Trianilino-(p-carbo-2'-ethyl-1'-hexyloxy) -1,3,5-triazin.

### Vorteilhafte wasserlösliche UVB-Filter sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bomylidenmethyl)sulfonsäure und ihre Salze sowie das 1,4-di(2-oxo-10-Sulfo-3-bornylidenmethyl)-Benzol und dessen Salze (die entprehenden 10-Sulfato-verbindungen, beispielsweise das entsprechende Natrium-, Kalium- oder Triethanolammonium-Salz), auch als Benzol-1,4-di(2-oxo-3-bornylidenmethyl-10-Sulfonsäure bezeichnet

Die Liste der genannten UVB-Filter, die in Kombination mit den erfindungsgemäßen Wirkstoffkombinationen verwendet werden können, soll selbstverständlich nicht limitierend sein.

Gegenstand der Erfindung ist auch die Verwendung einer Kombination der erfindungsgemäß verwendeten Biochinone mit mindestens einem UVB-Filter als Antioxidans bzw. die Verwendung einer Kombination der erfindungsgemäß verwendeten Biochinone mit mindestens einem UVB-Filter als Antioxidans in einer kosmetischen oder dermatologischen Zubereitung zur Anwendung am Haar.

Es kann auch von Vorteil sein, die erfindungsgemäß verwendeten Biochinone mit UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'tert.Butylphenyl)-3-(4'-methoxyphenyl)propan-1,3-dion und um 1-Phenyl-3-(4'isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel, Siliconderivate und/oder Kammpolymere.

In kosmetischen Zubereitungen zur Festigung der Haare, wie z.B. Haarsprays, Haarlacke, Schaumfestiger, Flüssigfestiger, Stylinggele usw., können die erfindungsgemäß einzusetzenden Kammpolymere vorzugsweise in Konzentrationen von 0,5 bis 30 Gewichtsprozent eingesetzt werden.

Die erfindungsgemäßen Zusammensetzungen zur Festigung der Haare können als Haarsprays oder Schaumaerosole vorliegen und die dafür üblichen und dem Stand der Technik entsprechenden Zusätze enthalten, sofern eine entsprechende Kompatibilität vorliegt. Dies sind beispielsweise weitere Lösungsmittel wie niedere Polyalkohole und deren toxikologisch verträglichen Ether und Ester, Weichmacher, leicht- und schwerflüchtige Silicone, leicht- und schwerflüchtige verzweigte bzw. unverzweigte Kohlenwasserstoffe, Emulgatoren, Antioxidantien, Wachse, Stabilisatoren, pH-Wert-Regulatoren, Farbstoffe, Konsistenzgeber, Antistatika, UV-Absorber, Parfums, usw.

Soll die erfindungsgemäße Zusammensetzung als Haarspray oder Schaumaerosol verwendet werden, so wird in der Regel ein Treibmittel zugesetzt. Übliche Treibmittel sind niedere Alkane, beispielsweise Propan, Butan oder Isobutan, Dimethylether, Stickstoff, Stickstoffdioxid oder Kohlendioxid oder Gemische aus diesen Substanzen.

Bei Verwendung in mechanischen Sprüh- oder Schaumvorrichtungen, beispielsweise Sprühpumpen oder manuellen Schaumpumpen bzw. Squeezesystemen, kann das Treibmittel in der Regel entfallen.

Bei kosmetischen und dermatologischen Zubereitungen gemäß der Erfindung beispielsweise kann es sich beispielsweise auch um Shampoonierungsmittel, Zubereitungen zum Fönen oder Einlegen der Haare, Zubereitungen zum Färben, um eine Frisier- oder Behandlungslotion handeln.

Erfindungsgemäße Zubereitungen können sich gegebenenfalls vorteilhaft durch einen Gehalt an Tensiden auszeichnen. Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und - je nach Wunsch - für Schaumregulierung.

Bei den hydrophilen Anteilen eines Tensidmoleküls handelt es sich meist um polare funktionelle Gruppen, beispielweise -COO-, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische Ionen. Kationische Tenside sind beinahe ausschließlich durch das Vorhandensein einer quarternären Ammoniumgruppe gekennzeichnet. In wäßriger Lösung bilden sie im sauren oder neutralen Milieu positiv geladene organische Ionen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

RNH₂⁺CH₂CH₂COOH X⁻ (bei pH=2) X⁻ = beliebiges Anion, z.B. Cl⁻

RNH₂⁺CH₂CH₂COO⁻ (bei pH=7)

RNHCH₂CH₂COO⁻ B⁺ (bei pH=12) B⁺ = beliebiges Kation, z.B. Na⁺

Typisch für nicht-ionische Tenside sind Polyether-Ketten. Nicht-ionische Tenside bilden in wäßrigem Medium keine Ionen.

### A. Anionische Tenside

Vorteilhaft zu verwendende anionische Tenside sind
Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoyl-hydrolysiertes Soja Protein und Natrium-/ Kalium Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. AcylLactylate, lauroyllactylat, Caproyllactylat
6. Alaninate
Carbonsäuren und Derivate, wie
1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6 Citrat und Natrium PEG-4 Lauramidcarboxylat,
3. Ether-Carbonsäuren, beispielsweise Natriumlaureth-13 Carboxylat und Natrium PEG-6 Cocamide Carboxylat,

Phosphorsäureester und Salze, wie beispielsweise DEA-Oleth-10-Phosphat und Dilaureth-4 Phosphat,
Sulfonsäuren und Salze, wie
1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat sowie
Schwefelsäureester, wie
1. Alkylethersulfat, beispielsweise Natrium-, Ammonium-, Magnesium-, MIPA-, TIPA- Laurethsulfat, Natriummyrethsulfat und Natrium C₁₂₋₁₃ Parethsulfat,
2. Alkylsulfate, beispielsweise Natrium-, Ammonium- und TEA- Laurylsulfat.

### B. Kationische Tenside

Gegebenenfalls vorteilhaft zu verwendende kationische Tenside sind
1. Alkylamine,
2. Alkylimidazole,
3. Ethoxylierte Amine und
4. Quaternäre Tenside.
5. Esterquats
Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxyliertel propoxylierte Ester, ethoxylierte/ propoxylierte Glycerinester, ethoxylierte/propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7 Polyglycerinester, Diglycerinester, Monoglycerinester
8. Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.

In der Regel ist im Sinne der vorliegenden Erfindung die Verwendung von anionischen, amphoteren und/oder nicht-ionischen Tensiden gegenüber der Verwendung von kationischen Tensiden bevorzugt.

Die kosmetischen und dermatologischen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Verdickungsmittel, Emulgatoren, Fette, Öle, Wachse, organische Lösungsmittel, Bakterizide, Parfüme, Farbstoffe oder Pigmente, deren Aufgabe es ist, die Haare oder die kosmetische oder dermatologische Zubereitung selbst zu färben, Elektroyte, Substanzen gegen das Fetten der Haare.

Unter Elektrolyten im Sinne der vorliegenden Erfindung sind wasserlösliche Alkali-, Ammonium-, Erdalkali- (unter Einbeziehung des Magnesiums) und Zinksalze anorganischer Anionen und beliebige Gemische aus solchen Salzen zu verstehen, wobei gewährleistet sein muß, daß sich diese Salze durch pharmazeutische oder kosmetische Unbedenklichkeit auszeichnen.

Die erfindungsgemäßen Anionen werden bevorzugt gewählt aus der Gruppe der Chloride, der Sulfate und Hydrogensulfate, der Phosphate, Hydrogenphosphate und der linearen und cyclischen Oligophosphate sowie der Carbonate und Hydrogencarbonate.

Kosmetische Zubereitungen, die ein Shampoonierungsmittel darstellen, enthalten vorzugsweise mindestens eine anionische, nicht-ionische oder amphotere oberflächenaktive Substanz, oder auch Gemische aus solchen Substanzen im wäßrigen Medium und Hilfsmittel, wie sie üblicherweise dafür verwendet werden. Die oberflächenaktive Substanz bzw. die Gemische aus diesen Substanzen können in einer Konzentration zwischen 1 Gew.-% und 50 Gew.-% in dem Shampoonierungsmittel vorliegen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält z. B. auch Kammpolymere.

Die erfindungsgemäßen Zusammensetzungen enthalten gegebenenfalls die in der Kosmetik üblichen Zusatzstoffe, beispielsweise Parfüm, Verdicker, Farbstoffe, Desodorantien, antimikrobielle Stoffe, rückfettende Agentien, Komplexierungs- und Sequestrierungsagentien, Perlglanzagentien, Pflanzenextrakte, Vitamine, Wirkstoffe und dergleichen.

Alle Mengenangaben, Anteile und Prozentanteile sind, soweit nicht anders angegeben, auf das Gewicht und die Gesamtmenge bzw. auf das Gesamtgewicht der Zubereitungen bezogen.

Die nachfolgenden Beispiele sollen die vorliegende Erfindung verdeutlichen. Es werden Gewichtsprozente angegeben.

CLA1 bedeutet in den folgenden Beispielen die folgende Fettsäure-CLA-Isomeren-Zubereitung:

**Tabelle 1:**

| CLA1 | |
|---|---|
| Fettsäure | Gew.-% |
| | |
| 16 : 0 | 6,9 |
| 18 : 0 | 2,5 |
| 18 : 1 | 15,3 |
| 18 : 2 | 0,8 |
| 18 : 2 (CLA) (Rest nicht definiert) | 73,8 a) |
| | |

| a) CLA-Zusammensetzung (- Octadiensäure) | |
|---|---|
| | |
| 9c, 11t/9t, 11c - | 34,6 |
| 10t, 12c- | 35,9 |
| | |
| 9c, 11c/10c, 12c - | 1,7 |
| 9t, 11t/10t, 12t - | 1,6 |

### Vergleichs - Beispiele 1 - 3

| Conditioner-Shampoo mit Perlglanz | | | |
|---|---|---|---|
| | **1** | **2** | **3** |
| Polyquaternium-10 | 0,5 | 0,5 | 0,5 |
| Natriumlaurethsulfat | 9,0 | 9,0 | 9,0 |
| Cocoamidopropylbetain | 2,5 | 2,5 | 2,5 |
| Perlglanzmittel | 2,0 | 2,0 | 2,0 |
| Coenzym Q10 | 0,3 | 0,03 | 3,5 |
| Minoxidil | 5,0 | 5,0 | 5,0 |
| Carnitin | - | 0,5 | - |
| CLA1 | - | - | 0,3 |
| Konservierungsmittel, Parfüm, Verdicker, pH-Einstellung und Lösungsvermittler | q.s. | q.s. | q.s. |
| Wasser, VES (vollentsalzt) | ad 100,0 | ad 100,0 | ad 100,0 |

Der pH-Wert wird auf 6 eingestellt.

### Vergleichs - Beispiele 4 - 6

| klares Conditioner-Shampoo | | | |
|---|---|---|---|
| | **4** | **5** | **6** |
| Polyquaternium-10 | 0,5 | 0,5 | 0,5 |
| Natriumlaurethsulfat | 9,0 | 9,0 | 9,0 |
| Cocoamidopropylbetain | 2,5 | 2,5 | 2,5 |
| Coenzym Q10 | 0,1 | 0,4 | 0,2 |
| Minoxidil | 7,0 | 7,0 | 7,0 |
| Liponsäure | 0,2 | - | - |
| Folsäure | - | 0,2 | - |
| Arginin | - | - | 1,0 |
| Konservierungsmittel, Parfüm, Verdicker, pH-Einstellung und Lösungsvermittler | q.s. | q.s. | q.s. |
| Wasser, VES | ad 100,0 | ad 100,0 | ad 100,0 |

Der pH-Wert wird auf 6 eingestellt.

### Vergleichs - Beispiele 7 - 9

| klares Light-Shampoo mit Volumeneffekt | | | |
|---|---|---|---|
| | **7** | **8** | **9** |
| Natriumlaurethsulfat | 10,0 | 10,0 | 10,0 |
| Cocoamidopropylbetain | 2,5 | 2,5 | 2,5 |
| Coenzym Q10 | 0,8 | 0,5 | 0,4 |
| Finasterid | 1,0 | 1,0 | 1,0 |
| Acetylcamitin | 1,0 | 0,1 | - |
| Dinatriumsuccinat | - | 1,0 | - |
| Liponsäure | - | - | 0,5 |
| Carnitin | - | - | 0,1 |
| Arginin | - | - | 0,2 |
| Konservierungsmittel, Parfüm, Verdicker, pH-Einstellung und Lösungsvermittler | q.s. | q.s. | q.s. |
| Wasser, VES | ad 100,0 | ad 100,0 | ad 100,0 |

Der pH-Wert wird auf 5,5 eingestellt.

### Vergleichs - Beispiele 10 - 13

| Haarspray | | |
|---|---|---|
| | **10** | **11** |
| Octylacrylamide/Acrylates/ Butylaminoethyl | | |
| Methacrylate Copolymer | 2,5 | 2,5 |
| Coenzym Q10 | 0,05 | 0,08 |
| Gammalinolensäure | 8,0 | 8,0 |
| 1 -(4'-tert. Butylphenyl)-3-(4'methoxy- | | |
| phenyl)propan-1,3-dion (Parsol 1789) | 1,0 | - |
| 2,4,6-Trianilino-(p-carbo-2'-ethyl-1'-hexyloxy)- | | |
| 1,3,5,-triazin | - | 1,0 |
| Ethanol abs. | 39,0 | 39,0 |
| Parfüm, Lösungsvermittler, Neutralisationsmittel/pH-Einstellung, Pflegestoffe | q.s. | q.s. |
| Dimethylether | ad 100 | ad 100 |

| | **12** | **13** |
|---|---|---|
| PVP/VA Copolymer | 8,0 | 8,0 |
| Coenzym Q10 | 0,01 | 0,05 |
| Zinkchlorid | 6,0 | 6,0 |
| 3-(4-Methylbenzyliden)-campher | 1,0 | - |
| 4-Methoxyzimtsäure-(2-ethylhexyl)-ester | - | 1,0 |
| Ethanol abs. | 39,0 | 39,0 |
| Parfüm, Lösungsvermittler, Pflegestoffe | q.s. | q.s. |
| Dimethylether | ad 100 | ad 100 |

### Vergleichs - Beispiele 14-16

| Haarkur | | | |
|---|---|---|---|
| | **14** | **15** | **16** |
| Hydroxypropylmethylcellulose | 0,5 | 0,5 | 0,5 |
| Cetrimoniumbromid | 1,0 | 1,0 | 1,0 |
| Glycerin | 3,0 | 3,0 | 3,0 |
| Cetearylalkohol | 2,5 | 2,5 | 2,5 |
| Glycerylstearat | 2,0 | 2,0 | 2,0 |
| Ubichinon Q10 | 0,02 | 0,0002 | 0,2 |
| Epicatechin | 4,0 | 4,0 | 4,0 |
| Carnitin | 2,0 | 0,4 | - |
| CLA1 | - | 0,5 | - |
| Liponsäure | - | 0,3 | 1,0 |
| Alphaglucosylrutin | - | 0,2 | - |
| Arginin | - | - | 1,5 |
| Konservierungsmittel, Parfüm, pH-Einstellung | q.s. | q.s. | q.s. |
| Wasser, VES (vollentsalzt) | ad 100,0 | ad 100,0 | ad 100,0 |

Der pH-Wert wird auf 3,5 eingestellt.

### Vergleichs - Beispiele 17-19

| Haarspülung | | | |
|---|---|---|---|
| | **17** | **18** | **19** |
| Behentrimoniumchlorid | 1,0 | 1,0 | 1,0 |
| Glycerin | 3,0 | 3,0 | 3,0 |
| Hydroxyethylcellulose | 0,2 | 0,2 | 0,2 |
| Cetearylalkohol | 3,0 | 3,0 | 3,0 |
| Ubichinon Q10 | 0,0004 | 0,05 | 0,5 |
| Epigallocatechin-3-gallat | 2,0 | 2,0 | 2,0 |
| Folsäure | 0,8 | - | - |
| Vitamin E | - | 0,2 | - |
| Dinatriumsuccinat | - | - | 1,0 |
| Konservierungsmittel, Parfüm, | | | |
| pH-Einstellung | q.s. | q.s. | q.s. |
| Wasser, VES | ad 100,0 | ad 100,0 | ad 100,0 |

Der pH-Wert wird auf 3,0 eingestellt.

### Vergleichs - Beispiele 20 und 21

| Schaumfestiger | | |
|---|---|---|
| | **20** | **21** |
| PVPNA Copolymer | 8,0 | 8,0 |
| Hydroxyethyl Cetyldimonium Phosphate | 0,1 | 0,1 |
| Coenzym Q10 | 0,07 | 0,01 |
| Pinacidil | 2,0 | 2,0 |
| Camitin | 0,1 | - |
| Arginin | - | 1,0 |
| Parfüm, Lösungsvermittler, Pflegestoffe | q.s. | q.s. |
| Ethanol abs. | 10,0 | 10,0 |
| Propan/Butan | 10,0 | 10,0 |
| Wasser, VES (vollentsalzt) | ad 100,0 | ad 100,0 |

### Beispiele 22 und 23

| | **22** | **23** |
|---|---|---|
| PVP/VA Copolymer | 5,0 | 5,0 |
| Polyquaternium-16 | 2.0 | 2.0 |
| Hydroxyethyl Cetyldimonium Phosphate | 0,1 | 0,1 |
| Coenzym Q10 | 0,0001 | 0,004 |
| Minoxidil | 2,0 | 2,0 |
| Finasterid | 1,0 | 1,0 |
| Carnitin | - | 1,0 |
| Arginin | - | 2,0 |
| Liponsäure | 0,2 | 0,5 |
| CLA1 | - | 1,0 |
| 1-(4'-tert. Butylphenyl)-3-(4'methoxy-phenyl)propan-1,3-dion (Parsol 1789) | 1,0 | 2,0 |
| 2,4,6-Trianilino-(p-carbo-2'ethyl-1'hexyloxy)-1,3,5-triazin | 1,0 | 2,0 |
| Parfüm, Lösungsvermittler, Pflegestoffe | q.s. | q.s. |
| Ethanol abs. | 10,0 | 10,0 |
| Propan/Butan | 10,0 | 10,0 |
| Wasser, VES | ad 100,0 | ad 100,0 |

### Beispiele 24 und 25

| Stylinggele | | |
|---|---|---|
| | **24** | **25** |
| PVP/VA Copolymer | 5,0 | 5,0 |
| Ceteareth-25 | 0,1 | 0,1 |
| Carbomer | 0,8 | 0,8 |
| Coenzym Q10 | 0,01 | 0,001 |
| Minoxidil | 1,0 | 1,0 |
| Finasterid | 1,0 | 1,0 |
| Gammalinolensäure | 4,0 | 4,0 |
| Acetylcamitin | 0,2 | - |
| Alphaglucosylrutin | 0,2 | - |
| CLA1 | - | 0,5 |
| Parfüm, Lösungsvermittler, Pflegestoffe Neutralisationsmittel/pH-Einstellung | q.s. | q.s. |
| Ethanol abs. | 10,0 | 10,0 |
| Wasser, VES | ad 100,0 | ad 100,0 |

### Vergleichs - Beispiel 26

| W/O-Creme | |
|---|---|
| | **Gew.-%** |
| Vaseline DAB 9 | 13,0 |
| Glycerin DAB 9 | 6,3 |
| Wasser VES | 34,4 |
| Paraffinöl (Mineralöl 5E®, Shell) | 43,2 |
| Cetearylalkohol/PEG-40-Castor Oil/Natriumcetearyl-sulfat (Emulgade® F, Henkel KGaA) | 2,5 |
| Coenzym Q10 | 0,6 |
| Minoxidil | 2,0 |

In die 75°C warme Fettphase werden 0,6 Teile Coenzym Q₁₀ in 3 Teilen Paraffinöl gelöst eingearbeitet Die Fettphase wird sodann zu der 75°C warmen Wasserphase gegeben, verrührt und homogenisiert bis eine gleichmäßige hellgelbe Creme entstanden ist

### Vergleichs - Beispiel 27

| W/O-Creme | |
|---|---|
| | **Gew.-%** |
| PEG-1 Glyceryl-Oleostearat + | |
| Paraffinwachs | 8,0 |
| Vaseline DAB | 2,8 |
| Paraffinwachs/Paraffin | 1,8 |
| Paraffinöl (Mineralöl 5E® Shell) | 11,5 |
| Ceresin | 2,2 |
| Octyldodecanol | 10,0 |
| Coenzym Q10 | 0,8 |
| Finasterid | 1,0 |
| Propylenglycol | 1,0 |
| Glycerin | 1,0 |
| Carnitin | 0,7 |
| Wasser VES | 59,4 |
| Summe Additive (Parfüm, Konservierung, Stabilisation | 0,8 |

In die 75°C warme Fettphase werden 0,8 Teile Coenzym Q₁₀ in 6 Teilen Paraffinöl gelöst eingearbeitet. Die Fettphase wird sodann zu der 75°C warmen Wasserphase gegeben, verrührt und homogenisiert, bis eine gleichmäßige hellgelbe Creme entstanden ist.

### Beispiel 28

| O/W-Creme | |
|---|---|
| | **Gew.-%** |
| Octyldodecanol (Eutanol® G,Henkel KGaA) | 9,3 |
| Cetearylalkohol/PEG-40-Castor-Oil/Natriumcetearylsulfat (Emulgade® F, Henkel KGaA) | 3,7 |
| Wasser VES | 72,7 |
| Glycerin DAB 9 | 4,6 |
| Paraffinöl (Mineralöl 5E®, Shell) | 7,7 |
| Coenzym Q10 | 0,9 |
| Minoxidil | 1,0 |
| Finasterid | 1,0 |
| Gammalinolensäure | 1,0 |
| Epicatechin-3-gallat | 2,0 |
| Arginin | 1,0 |

In die 75°C warme Fettphase werden 0,9 Teile Coenzym Q₁₀ in 4 Teilen Paraffinöl gelöst eingearbeitet Die Fettphase wird sodann zu der 75°C warmen Wasserphase gegeben, verrührt und homogenisiert, bis eine gleichmäßige hellgelbe Creme entstanden ist.

### Vergleichs - Beispiel 29

| O/W-Lotion | |
|---|---|
| | Gew.-% |
| Steareth-2 | 3,0 |
| Steareth-21 | 2,0 |
| Cetearylalkohol/PEG-40-Castor-Oil/Natriumcetearylsulfat (Emulgade® F, Henkel KGaA) | 2,5 |
| Paraffinöl (Mineralöl 5E®, Shell) | 14,4 |
| Propylenglycol | 1,0 |
| Coenzym Q10 | 0,1 |
| Minoxidil | 2,0 |
| Folsäure | 0,9 |
| Glycerin | 1,0 |
| Wasser VES | 74,3 |
| Summe Additive (Parfum, Konservierung, Stabilisation) | 0,8 |

In die 75°C warme Fettphase werden 0,1 Teile Coenzym Q₁₀ in 5,2 Teilen Paraffinöl gelöst eingearbeitet. Die Fettphase wird sodann zu der 75°C warmen Wasserphase gegeben, verrührt und homogenisiert, bis eine gleichmäßige hellgelbe Lotion entstanden ist.

### Vergleichs - Beispiel 30

| O/W-Lotion | |
|---|---|
| | **Gew.-%** |
| Octyldodecanol(Eutanol® G, Henkel KGaA) | 5,6 |
| Cetearylalkohol/PEG-40-Castor-Oil/Natriumcetearylsulfat (Emulgade®F, Henkel KGaA) | 8,9 |
| Cetearylisononanoat | |
| (Cetiol®5N, Henkel KGaA) | 7,5 |
| Wasser VES | 62.3 |
| Glycerin DAB 9 | 4,7 |
| Paraffinöl (Mineralöl 5E®, Shell) | 10,0 |
| Coenzym Q10 | 0,4 |
| Zinkchlorid | 8,0 |
| Dinatriumsuccinat | 0,6 |

In die 75°C warme Fettphase werden 0,4 Teile Coenzym Q₁₀ in 6 Teilen Paraffinöl gelöst eingearbeitet. Die Fettphase wird sodann zu der 75°C warmen Wasserphase gegeben, verrührt und homogenisiert, bis eine gleichmäßige hellgelbe Lotion entstanden ist.

### Vergleichs - Beispiel 31

| Öl | |
|---|---|
| | **Gew.-Teile** |
| Glyceryltricaprylat | |
| (Miglyol® 812, Dynamit Nobel) | 21,0 |
| Hexyllaurat (Cetiol® A, Henkel KGaA) | 20,0 |
| Octylstearat (Cetiol® 886, Henkel KGaA) | 20,0 |
| Paraffinöl (Mineralöl 5®, Shell) | 35,0 |
| CLA1 | 2,0 |
| Coenzym Q9 | 1,6 |
| Coenzym Q10 | 0,4 |
| Minoxidil | 1,0 |
| Gammalinolensäure | 8,0 |

Die Komponenten werden bei 25°C verrührt, bis eine gleichmäßige, klare Mischung entstanden ist.

### Vergleichs - Beispiel 32

| Haarwasser | |
|---|---|
| | **Gew.-%** |
| Minoxidil | 2,0 |
| Coenzym Q10 | 1,0 |
| Ethanol | 10,0 |
| Wasser | 89,0 |

Die Bestandteile werden vermischt und gelöst.

## Patentansprüche

1. Verwendung
a) einer Verbindung oder mehrerer Verbindungen aus der Gruppe der Biochinone in Kombination mit
b) einer Verbindung oder mehreren Verbindungen aus der Gruppe der Kaliumkanalöffner und in Kombination mit
c) einer Verbindung oder mehrerer Verbindungen aus der Gruppe der 5-alpha-Reduktasehemmer,
zur Herstellung kosmetischer oder dermatologischer Zubereitungen zur Behandlung und Prophylaxe von Kopfschuppen.

2. Verwendung nach Anspruch 1, **dadurch gekennzeichnet, dass** man zusätzlich eine oder mehrere Verbindungen aus der Gruppe bestehend aus Carnitin, Arginin, Bernsteinsäure, Folsäure, konjugierten Fettsäuren, jeweils deren Derivaten, und Antioxidantien verwendet.

3. Verwendung nach den Ansprüchen 1 und 2, **dadurch gekennzeichnet, dass** die Kombinationen folgende Verbindungen umfassen:
a) Ubichinone, insbesondere Coenzym Q-10, und Minoxidil, oder
b) Ubichinone, insbesondere Coenzym Q-10, und Finasterid und/oder Gammalinolensäure, oder
c) Ubichinone, insbesondere Coenzym Q-10, und Minoxidil und Finasterid und/oder Gammalinolensäure.

4. Kosmetische oder dermatologische Zubereitung, **dadurch gekennzeichnet, dass** sie eine Kombination
a) einer Verbindung oder mehrerer Verbindungen aus der Gruppe der Biochinone mit
b) einer Verbindung oder mehrerer Verbindungen aus der Gruppe der Kaliumkanalöffner und mit
c) einer Verbindung oder mehrerer Verbindungen aus der Gruppe der 5-alpha-Reduktasehemmer
enthält.

5. zubereitung nach Anspruch 4, **dadurch gekennzeichnet, dass** sie zusätzlich eine oder mehrere Verbindungen aus der Gruppe bestehend aus Carnitin, Arginin, Bernsteinsäure, Folsäure, konjugierten Fettsäuren, jeweils deren Derivaten, und Antioxidantien enthält.

6. Zubereitung nach Anspruch 4, **dadurch gekennzeichnet, dass** die Kombinationen folgende Verbindungen umfassen:
a) Ubichinone, insbesondere Coenzym Q-10, und Minoxidil, oder
b) Ubichinone, insbesondere Coenzym Q-10, und Finasterid und/oder Gammalinolensäure, oder
c) Ubichinone, insbesondere Coenzym Q-10, und Minoxidil und Finasterid und/oder Gammalinolensäure.

## Claims

1. Use
a) of one or more compounds from the group of bioquinones in combination with
b) one or more compounds from the group of potassium channel openers and in combination with
c) one or more compounds from the group of 5-alpha-reductase inhibitors
for the production of cosmetic or dermatological preparations for treatment and prophylaxis of dandruff.

2. Use according to Claim 1, **characterized in that** one or more compounds from the group consisting of carnitine, arginine, succinic acid, folic acid, conjugated fatty acids, their derivatives in each case, and antioxidants are additionally used.

3. Use according to Claims 1 and 2, **characterized in that** the combinations comprise the following compounds:
a) ubiquinones, in particular coenzyme Q-10, and minoxidil, or
b) ubiquinones, in particular coenzyme Q-10, and finasteride and/or gamma-linolenic acid, or
c) ubiquinones, in particular coenzyme Q-10, and minoxidil and finasteride and/or gamma-linolenic acid.

4. Cosmetic or dermatological preparation, **characterized in that** it comprises a combination
a) of one or more compounds from the group of bioquinones with
b) one or more compounds from the group of potassium channel openers and with
c) one or more compounds from the group of 5-alpha-reductase inhibitors.

5. Preparation according to Claim 4, **characterized in that** it additionally comprises one or more compounds from the group consisting of carnitine, arginine, succinic acid, folic acid, conjugated fatty acids, their derivatives in each case, and antioxidants.

6. Preparation according to Claim 4, **characterized in that** the combinations comprise the following compounds:
a) ubiquinones, in particular coenzyme Q-10, and minoxidil, or
b) ubiquinones, in particular coenzyme Q-10, and finasteride and/or gamma-linolenic acid, or
c) ubiquinones, in particular coenzyme Q-10, and minoxidil and finasteride and/or gamma-linolenic acid.

## Revendications

1. Utilisation
a) d'une liaison ou de plusieurs liaisons du groupe des bioquinones en combinaison avec
b) une liaison ou plusieurs liaisons du groupe des activateurs de canal potassique et en combinaison avec
c) une liaison ou plusieurs liaisons du groupe des inhibiteurs des 5-alpha réductases,
pour la fabrication de préparations cosmétiques ou dermatologiques conçues pour le traitement et la prophylaxie des pellicules dans les cheveux.

2. Utilisation selon la revendication 1, **caractérisée en ce qu'**on utilise en outre une ou plusieurs liaisons du groupe comprenant la carnitine, l'arginine, l'acide succinique, l'acide folique, les acides gras conjugués et leurs dérivés et antioxydants respectifs.

3. Utilisation selon les revendications 1 et 2, **caractérisée en ce que** les combinaisons comprennent les liaisons suivantes :
a) Une ubiquinone, en particulier le coenzyme Q-10 et le minoxidil ou
b) Une ubiquinone, en particulier le coenzyme Q-10 et le finasteride et/ou l'acide gamma-linolénique, ou
c) Une ubiquinone, en particulier le coenzyme Q-10 et le minoxidil et le finasterid et/ ou l'acide gamma-linolénique.

4. Préparation cosmétique ou dermatologique **caractérisée en ce qu'**elle contient une combinaison
a) une liaison ou plusieurs liaisons du groupe des bioquinones avec
b) une liaison ou plusieurs liaisons du groupe des activateurs de canal potassique et avec
c) une liaison ou plusieurs liaisons du groupe des inhibiteurs des 5-alpha réductases.

5. Préparation selon la revendication 4, **caractérisée en ce qu'**elle contient en outre une ou plusieurs liaisons du groupe comprenant la carnitine, l'arginine, l'acide succinique, l'acide folique, les acides gras conjugués et leurs dérivés et antioxydants respectifs.

6. Préparation selon la revendication 4, **caractérisée en ce que** les combinaisons contiennent les liaisons suivantes :
a) Une ubiquinone, en particulier le coenzyme Q-10 et le minoxidil ou
b) Une ubiquinone, en particulier le coenzyme Q-10 et le finasteride et/ou l'acide gamma-linolénique, ou
c) Une ubiquinone, en particulier le coenzyme Q-10 et le minoxidil et le finasteride et / ou l'acide gamma-linolénique.
